# EUROPEAN PATENT APPLICATION

(11) **EP 3 920 189 A1**
(43) Date of publication of application: **08.12.2021**
(21) Application number: 20178230.7
(22) Date of filing: 04.06.2020
(51) Int. Cl.: G16H 30/20, G16H 40/63

(54) **ESTIMATING A POSITION OF AN ENDOSCOPE IN A MODEL OF THE HUMAN AIRWAYS**

(71) Applicant: Ambu A/S, 2750 Ballerup (DK)
(72) Inventor: JØRGENSEN, Andreas Härstedt, 2750 Ballerup (DK); SONNENBORG, Finn, 2750 Ballerup (DK); YU, Dana Marie, 2750 Ballerup (DK); LASSEN, Lee Herluf Lund, 2750 Ballerup (DK)
(74) Representative: AWA Denmark A/S

(57) **Abstract**

Disclosed is an image processing device for estimating a position of an endoscope in a model of the human airways using a machine learning data architecture trained to determine a set of anatomic reference positions, said image processing device comprising a processing unit operationally connectable to an image capturing device of the endoscope, wherein the processing unit is configured to obtain a stream of recorded images; continuously analyse the recorded images of the stream of recorded images using the machine learning data architecture to determine if an anatomic reference position of a subset of anatomic reference positions, from the set of anatomic reference positions, has been reached; and where it is determined that the anatomic reference position has been reached, update the endoscope position based on the anatomic reference position, and an endoscope system comprising an endoscope and an image processing device, a display unit comprising an image processing device, and a computer program product.

## Description

### Field

The present disclosure relates to an image processing device for estimating a position in a model of the human airways, an endoscope system comprising an endoscope and an image processing device, and a computer program product.

### Background

Examination of human airways with an endoscope, such as a bron-choscope, may be carried out to determine whether a patient has a lung disease, a tumor, a lung infection, or the like, and in some cases samples may be taken/removed from or inserted into part of the airways. The endoscope typically comprises an image capturing device, such as a camera, at a distal end of the endoscope to be inserted into the patient and connected to a display so as to provide the medical personnel with a view of the part of the airways, in which the distal end of the endoscope is positioned.

Typically, when an examination of the human airways is carried out, the medical personnel will need to search through most or all parts of the lung tree, such as trachea, the left and right bronchus and their respective bronchioles and alveoli, to check for any abnormalities. The information about the various parts is typically journalised for documentation purposes. Alternatively, in other cases, an investigation of a specific part of the human airways, such as a specific bronchiole is desired, for instance based on a magnetic resonance (MR) or computed topography (CT) scan result.

When navigating through the parts of the human airways, the medical personnel, however, often rely on experience to navigate the endoscope through the human airways, e.g. to reach most/all parts of the lung tree, and/or the specific part, based on the camera image from the endoscope. Since parts of the human airways, such as various bronchi or various bronchioles, often look rather similar, there is a risk of mistakes, e.g. in that the desired parts of the human airways are not reached or in that a part of the airways are mistaken for a different part in the airways. This, in turn, increases a risk that the patient is not properly examined.

In some devices/systems, further devices, such as echo devices, are used to determine the position of the distal end of the endoscope, which, however, increases the complexity of the examination for the medical personnel by introducing a further device to be controlled as well as increase the costs of the examination.

Often, it is difficult to document afterwards that a correct examination, e.g. a full examination of most or all parts of the human airways and/or an examination of a specific part of the human airways, has been carried out.

Thus, it remains a problem to provide an improved device/system for estimating a position of an endoscope in a model of the human airways.

### Summary

According to a first aspect, the present disclosure relates to an image processing device for estimating a position of an endoscope in a model of the human airways using a machine learning data architecture trained to determine a set of anatomic reference positions, said image processing device comprising a processing unit operationally connectable to an image capturing device of the endoscope, wherein the processing unit is configured to:
obtain a stream of recorded images;
continuously analyse the recorded images of the stream of recorded images using the machine learning data architecture to determine if an anatomic reference position of a subset of anatomic reference positions, from the set of anatomic reference positions, has been reached; and
where it is determined that the anatomic reference position has been reached, update the endoscope position based on the anatomic reference position.

Thereby, the image processing device may determine a position of the endoscope in the model of the human airways in a simple manner by analysing the stream of recorded images. Thereby an easier examination of the human airways may be provided for by allowing the medical personnel to focus on identifying abnormalities in the images from the endoscope rather than keeping track of the position of the endoscope. By the image processing device determining the position of the endoscope based on the recorded images, a need for additional devices, such as echo (e.g. ultrasound) devices or devices for electromagnetic navigation, may be eliminated, in turn allowing for a simpler examination for the medical personnel as well as a reduced amount of equipment.

This may, in turn, provide for an indication to the medical personnel of the position of the endoscope of the human airways, allowing the medical personnel to navigate through the human airways in an easy manner. Moreover, the risk of wrongful navigation, such as a navigation of the endoscope to a non-desired part of the airways, may be reduced by having an updated endoscope position, again reducing the risk that a desired part of the human airways is not examined due to wrongful navigation and/or a human procedural error by the medical personnel.

When updating the endoscope position, the previous position may be stored, e.g. at a storage medium, a computer, a server, or the like, allowing for an easy documentation that a correct examination of the human airways has been performed. For instance, it may be registered that the endoscope has been positioned in specific bronchioles of the right bronchus, in turn allowing for an easy documentation of the examination.

A model of the human airways may represent the airways of a person, such as the person, on which an examination is performed. For instance, the model may represent the general structure of the human airways, e.g. trachea, bronchi, bronchioles, and/or alveoli. The model may represent the human airways schematically, and/or may represent the specific structure of the person, on which the examination is performed. The model may be configured such that a determined position of the endoscope in the model substantially corresponds to, corresponds to, or is an actual position of the endoscope in the human airways of a person. The determined position and/or the actual position may correspond to or be a part or segment of the human airways, in which the endoscope is determined to be.

An anatomic reference position may be any position within the human airways, e.g. any position within the lung tree. An anatomic reference position may be and/or may correspond to a position, which the endoscope such as a distal end thereof, can take in the human airways. In some embodiments, an anatomic reference position is a position at which a visual characteristic occurs, which allows the image processing device to estimate, based on the image, the position of the endoscope. Alternatively or additionally, an anatomic reference position may be a position, at which a furcation occurs in the human airways, such as where the trachea bifurcates into the left and right bronchus, and/or where bronchi and/or bronchioles furcate.

In some embodiments, an anatomic reference position is, and/or corresponds to, a predetermined position in the model. An anatomic reference position may alternatively or additionally correspond to a plurality of predetermined positions in the model.

A set of anatomic reference positions may comprise two or more anatomic reference positions. In some embodiments, a subset of anatomic reference positions comprises two or more anatomic reference positions from the set of anatomic reference positions, such as some but not all of the anatomic reference positions from the set of anatomic reference positions. The subset of anatomic reference positions may be determined based on the endoscope position, such as a previously estimated endoscope position.

In some embodiments, the model comprises a number of predetermined positions in the model. A predetermined position in the model may corresponds to an anatomic reference position, potentially a unique anatomic reference position. In some embodiments, the model comprises a plurality of predetermined positions, each corresponding to one or more anatomic reference positions. A predetermined position may, for example, be a position in the trachea, a position in a right bronchus, a position in a left bronchus, a position in a secondary bronchus, a position in a bronchiole, a position in an alveolus, a position at a furcation between one or more of these, or any combination thereof. The predetermined position may be a position, which the endoscope can be estimated to be at.

The predetermined positions and/or an anatomic reference position may be one or more of: the vocal cords, trachea, right main bronchus, left main bronchus, and any one or more furcations occurring in the bronchi, such as bi- or trifurcations into e.g. secondary or tertiary bronchi, bronchioles, alveoli, or the like.

Throughout this text the terms "main bronchus" and "primary bronchus" may be used interchangeably.

Throughout this text, it will furthermore be appreciated that a "position" need not be restricted to a specific point but may refer to an area in the human airways, a portion of a part of the human airways, or a part of the human airways.

In some embodiments, the endoscope position may be determined based on one or more images from the image stream in combination with additional information, such as a previous position of the endoscope and/or information that the examination has recently begun. Alternatively, or additionally, the image processing device may provide a confidence score of the estimated position. When the confidence score is below a predetermined and/or adjustable confidence threshold, the image processing device may store this information, provide an indication that the confidence score is below the confidence score, provide an indication of one or more potential estimated positions of the endoscope, and/or ask for user input to verify an estimated position, potentially from one or more potential estimated positions.

In some embodiments, the endoscope position may be estimated as one of the plurality of predetermined positions present in the model which has the smallest distance to the anatomic reference position. Each of the anatomic reference positions may, in some embodiments, correspond to a predetermined position present in the model. In this case, the endoscope position may be determined as the one of the predetermined positions corresponding to the anatomic reference position, which has been reached.

The determination of whether an anatomic reference position has been reached may be performed by means of feature extraction from one or more images in the stream of images and using the machine learning data architecture on the extracted features of the image. Any known method of feature extraction may be used.

The machine learning data architecture may be any known machine learning data architecture. For example, the machine learning data architecture may comprise an artificial neural network, a Kalman-filter, a deep learning algorithm, or the like. The machine learning data architecture may be configured to include images of a determined anatomic reference position and/or features thereof in a dataset for use in training and/or further training of the machine learning data architecture.

The machine learning data architecture may be a deep-learning data architecture.

In some embodiments, a user may be able to correct a detection of an anatomic reference position and/or an updated position. The machine learning data architecture may be configured to include the corrected detection of the reaching of the anatomic reference position and/or the corrected updated position into a data set thereof, thereby allowing for the machine learning data architecture to be further trained. Alternatively or additionally, the machine learning data architecture may, where a subsequent detection of the reaching of an anatomic reference position results in the conclusion that a previously determination of the reaching of an anatomic reference position may have been erroneous, correct the position updated based on the erroneous determination and/or include the corrected determination and the image and/or features thereof into a dataset, potentially a training data set, of the machine learning data architecture.

The model of the human airways may be an overall and/or general model of the human airways, such as a schematic overview of the human airways including the lung tree and the trachea. The model may be provided as input specifically prior to each examination or may be an overall model used for most or all examinations. In some embodiments, the model may be a simplified model of the human airways. The model may, alternatively or additionally, be updated during the examination, e.g. in response to updating an endoscope position in the model. Alternatively or additionally, the model may be provided by means of results of a CT scan taken prior to the examination and/or updated subsequently using results of a CT scan taken prior to examination.

In some embodiments, the method further comprises displaying to a user the model. Throughout this disclosure, it will be appreciated that displaying a model may be or may comprise displaying a view of the model. The method may furthermore comprise indicating on the displayed model, a position of the endoscope and/or indicating on the displayed model an updated position of the endoscope. The indication of the position of the endoscope on the model may be a display of a segment of the human airways, in which the endoscope is estimated to be positioned, such as in a given bronchus and/or a given bronchiole. The indication may be carried out as a graphic indication, such as a coloured mark, a highlighted portion, a flashing portion, an overlay of a portion, or the like. The position may in some embodiments indicate a portion or segment of the airways, in which the endoscope is estimated to be positioned. The model and/or the indication of the endoscope position may be displayed on a display separate from and connected to or integrated with the image processing device. Alternatively, or additionally, indications of one or more previous positions may be displayed, potentially in combination with the indication of the endoscope position.

The processing unit of the image processing device may be any processing unit, such as a central processing unit (CPU), a graphics processing unit (GPU), a microcontroller unit (MCU), a field-programmable gate array (FPGA), or any combination thereof. The processing unit may comprise one or more physical processors and/or may be combined by a plurality of individual processing units.

The term "endoscope" may be defined as a device suitable for examination of natural and/or artificial body openings, e.g. for exploration of a lung cavity. Additionally, or alternatively, the term "endoscope" may be defined as a medical device.

In this specification, a proximal-distal direction may be defined as an axis extending along the parts of the insertion tube of the endoscope. Adhering to the definition of the terms distal and proximal, i.e. proximal being the end closest to the operator and distal being the end remote from the operator. The proximal-distal direction is not necessarily straight, for instance, if an insertion tube of the endoscope is bent, then the proximal-distal direction follows the curvature of the insertion tube. The proximal-distal direction may for instance be a centre line of the insertion tube.

The stream of recorded images may be a video stream. The stream of recorded images may be provided by an image capturing device, potentially arranged in the endoscope, such as in and/or at a tip part of the endoscope. The camera module may be configured to obtain a stream of images, representing the surroundings of a tip part of the endoscope.

The image processing device may estimate a position of a tip part of the endoscope, arranged at a distal part of the endoscope. The endoscope tip part may form and/or comprise a distal end of the endoscope. Alternatively or additionally, the image processing device may estimate a position of a camera module of the tip part or a distal lens or window, e.g. where a camera module is arranged proximally of the tip part.

The endoscope may further comprise one or more of a handle at a proximal end of the endoscope, a tip part at a distal end of the endoscope, an insertion tube extending from a proximal end to a distal end of the endoscope, and a bending section which may have a distal end segment which may be connected to a tip part. This may allow for the tip part to be manoeuvred inside the human airways.

The bending section may comprise a number of hingedly interconnected segments including a distal end segment, a proximal end segment, and a plurality of intermediate segments positioned between the proximal end segment and the distal end segment. At least one hinge member may interconnect adjacent segments with each other. The bending section may be a section allowing the tip part assembly to bend relative to an insertion tube, potentially so as to allow an operator to manipulate the tip part assembly while inserted into a body cavity of a patient. The bending section may be moulded in one piece or may be constituted by a plurality of moulded pieces.

In some embodiments, the subset comprises a plurality of anatomic reference positions.

Thereby, the image processing device may be able to determine whether one in a plurality of anatomic reference positions have been reached and consequently determine an updated endoscope position where multiple endoscope positions are possible when the endoscope is moved from a previous position thereof.

The subset may comprise some or all of the anatomic reference positions of the set of anatomic reference positions. In some embodiments, the subset comprises a predefined plurality of anatomic reference positions. Alternatively, or additionally, the subset may be selected by means of a machine learning data architecture, such as the machine learning data architecture that determines whether an anatomic reference position has been reached, or a second machine learning data architecture.

In some embodiments, the processing unit is further configured to:
where it is determined that the anatomic reference position has been reached, update the subset of anatomic reference positions.

This, in turn, allows for the subset to comprise possible anatomic reference positions, i.e. anatomic reference positions which the endoscope can reach from its current estimated position. Consequently, the image processing device may look only for a number of possible anatomic reference positions, in turn allowing for an increased computational efficiency and error robustness of the system.

The subset may be updated dynamically prior to, subsequent to or simultaneously with the updating of the endoscope position. The updated subset may be different from the subset. Alternatively or additionally, the updated subset may comprise a plurality of anatomic reference positions of the set of anatomic reference positions.

In some embodiments, the processing unit is configured to determine a second subset of anatomic reference position, where it is determined that the anatomic reference position, potentially from a first subset of anatomic reference positions, has been reached. Additionally or alternatively, a first subset may initially be determined and where it is determined that an anatomic reference position of the first subset has been reached, a second subset may be determined.

The updated and/or the second subset may comprise the same number of anatomic reference positions as the subset and/or the first subset. Alternatively, the updated and/or second subset may comprise fewer or more anatomic reference positions than the subset and/or the first subset.

In some embodiments, the subset may be updated and/or the second subset may be generated based on the reached anatomic reference position and/or based on an estimated position of the endoscope.

In some embodiments, the updated subset of anatomic reference positions comprises at least one anatomic reference positions from the subset of anatomic reference positions.

This, in turn, allows the image processing device to determine a backwards movement of the endoscope, such as a movement of the endoscope to a previous position thereof.

The at least one anatomic reference positions from the subset of anatomic reference positions may be or may comprise the reached anatomic reference position. Alternatively or additionally, the at least one anatomic reference positions from the subset of anatomic reference positions may be or may comprise a plurality of previously reached anatomic reference positions.

In some embodiments, the anatomic reference position is a branching structure comprising a plurality of branches. The image processing device may be further configured to: determine which branch from the plurality of branches the endoscope enters; and update the endoscope position based on the determined branch.

Thereby, the risk that a wrong endoscope position is estimated where anatomic reference positions look similar may be reduced. This moreover allows for an improved registration of, in which part of the airways the endoscope has been, e.g. so as to make sure that a sufficiently detailed examination has been performed. For example, where branchings in the left and right main bronchus, respectively, may look similar, the image processing device may estimate the endoscope position being aware of, whether the endoscope has entered the left or right main bronchus. Hence, the image processing device may be able to distinguish between, for instance, furcations into secondary bronchi in the right and left primary bronchi, respectively.

The branching may be a furcation, such as a bifurcation, a trifurcation, or the like. The image processing device may determine which branch from the plurality of branches, the endoscope enters by analysing the image stream. The determined branch may be the branch, which the endoscope enters. Alternatively, or additionally, the image processing device may determine which branch the endoscope enters based on input from one or more sensors, such as a compass or an accelerometer, potentially arranged at the handle of the endoscope, magnetic resonance devices, or the like. In some embodiments, the image processing device may use a machine learning data architecture to identify the branching and/or to determine which branch from the plurality of branches, the endoscope enters.

Where the stream of images is provided to the operator and/or medical personnel, e.g. via a display unit, the image processing device may further be able to indicate to the operator and/or medical personnel the branching. In some embodiments, each of the branches may be indicated. The branching and/or branches may be graphically indicated, e.g. by means of a graphic overlay, such as a text and/or colour overlay, on an image of the stream.

In some embodiments, the indications of the branching and/or branches may be indicated upon request from a user, e.g. medical personnel. In other words, the user may activate an indication of the branching and/or branches. The request may, for instance, be input to the image processing device by means of a button push, a touch screen push, and/or a voice command. Hence, the branching and/or specific branches may be indicated on an endoscope image to assist the user in navigating the endoscope, when the user wishes so. Hence, where the user does not need navigating assistance, the indications need not be provided.

In some embodiments, the processing unit is further configured to:
where it is determined that the anatomic reference position has been reached, storing a part of the stream of recorded images.

This, in turn, allows for an improved documentation of the examination as it may subsequently be verified that the desired part of, such as all of, the human airways has been examined. Alternatively or additionally the video stream may subsequently be (re-)checked for abnormalities at respective positions in the airways.

The part(s) of the stream may be stored on a local storage space, preferably a storage medium. The stream may alternatively or additionally be transmitted to an external device, such as a computer, a server, or the like. In some embodiments, the stored stream of recorded images may be used to aid the system in determining the reaching of the specific anatomic reference position.

In some embodiments, the recorded image stream may be stored with a label relating the video stream to the reached anatomic reference position and/or to the estimated endoscope position determined based on the reached anatomic reference position. The label may, for instance, be in the shape of metadata, an overlay, a storage structure, a file name structure of a file comprising the recorded image stream, or the like.

In some embodiments, a user, such as medical personnel, may subsequently correct an endoscope position determined by the image processing device based on the stored recorded image stream. A corrected endoscope position may be transmitted to the image processing device, potentially introducing one or more images from the stored recorded image stream and/or the anatomic reference position in a training dataset so as to allow the machine learning data architecture to be trained.

In some embodiments, the processing unit is further configured to:
prior to the step of updating the subset of anatomic reference positions, generate the model of the human airways, and/or
subsequent to the step of updating the subset of anatomic reference positions, update the model of the human airways based on the reached anatomic reference position and/or an anatomic reference position of the updated subset of anatomic reference positions.

Thereby, the model may be updated according to information from the examination, such as according to the physiology of the individual patient, in turn allowing for an improved view of the airways of the individual patient to the medical personnel. For instance, where a bifurcation is missing in the airways of a patient, this may be taken into account in the model by using the information from the examination.

Updates of the model may, for example, consist of or comprise addition of a modelled part of the human airways, removal of a modelled part of the human airways from the model, selection of a part of the model of the human airways, and/or addition and/or removal of details in the model. For example, where it is determined that the endoscope is in the right bronchus, certain bronchi and/or bronchioles of the right bronchus may be added to the model.

The model may be updated to show further parts of the airways, e.g. in response to the detection thereof. Detection of further parts may be performed in response to and/or as a part of determining whether an anatomic reference position has been reached. The detection of further parts may be based on the stream of images. The detection may be carried out by the machine learning data architecture. For example, where e.g. a furcation indicating bronchioles is detected in the stream of images, the model may be updated to indicate these bronchioles. The location of the detected parts may be estimated based on the position of the endoscope.

Additionally or alternatively, the model may be updated according to the reached anatomic reference position. The model may be updated based on the stream of recorded images.

In some embodiments, the displayed model, i.e. a view of the model, may be updated and/or the display and/or view of the model may be updated. The display of the model may be updated to show a section of the model, e.g. a zoomed in section of the model.

In some embodiments, the model is created as and/or is based on a general well-known model of the human airways. The model may be a schematic structure of the human airways.

The endoscope position may be mapped to the model. In some embodiments, updating the endoscope positions comprise selecting one position from a plurality of predetermined positions of the model. The selection of the position may comprise selecting a position which is nearest and/or best approximates the endoscope position. The selection may be based on one or more images from the stream of images.

In some embodiments, the model of the human airways is a schematic model of the human airways, preferably generated based on images from a magnetic resonance (MR) scan output and/or a computed tomography (CT) scan output.

By providing a model specific for each patient, the accuracy of the estimation of position may be improved as specific knowledge of the specific airways is provided.

The MR scan output and/or the CT scan output may be converted into a potentially simplified schematic model. In some embodiments, the additional knowledge of the human airways, such as an overall model thereof, may be used in combination with the MR scan output and/or the CT scan output to provide the schematic model of the human airways.

The MR and/or CT scan output may be unique for a patient. Potentially the model may be generated based on a number of MR and/or CT scan outputs from the same or from different humans.

In some embodiments, a model of the human airways can be generated and/or extracted from the MR and/or CT scan output.

In some embodiments, the processing unit is further configured to:
subsequent to the step of updating the endoscope position, perform a mapping of the endoscope position to the model of the human airways and display the endoscope position on a view of the model of the human airways.

By mapping the endoscope position to the model of the human airways may here be understood that the endoscope position may be determined in relation to or relative to the model of the human airways. In some embodiments, the mapping comprises determining a part of the human airways, in which the endoscope is positioned. Alternatively or additionally the mapping may comprise determining a position in the model from a plurality of positions in the model which corresponds to the endoscope position. In some embodiments, the endoscope position may be mapped to a position in the model from a plurality of positions in the model which is nearest amongst the plurality of positions to the endoscope position.

The view of the model of the human airways may be a two-dimensional view, such as a two-dimensional schematic view schematically showing the human airways. A two-dimensional schematic view may for example show a cross-section of the human airways, e.g. in the shape of a lung tree. Alternatively or additionally, the view of the model may be provided so as to show or indicate a third dimension, e.g. by providing a plurality of two-dimensional views, such as two cross-sections, and/or by allowing a rotation of two-dimensional view 180 degrees, up to 360 degrees, or 360 degrees around a rotational axis. Where a third dimension is to be indicated, a rotation, potentially up 360 degrees or 360 degrees, about each of three axes, i.e. the x-, y-, and z-axes, may be provided.

The view may be displayed on a display unit potentially comprising an electronic display and/or a monitor, e.g. a flat-panel display (FPD), such as a liquid crystal display (LCD), a light-emitting diode (LED) display, or the like. In some embodiments the electronic display may be a touchscreen. In some embodiments, the display unit comprises the image processing device.

The endoscope position may be indicated on the display in any known way. For example, the position may be indicated by a part of the human airways, in which the endoscope position is located, changing colour, flashing, being highlighted, or the like, by a marking, such as a black and white or coloured shape, e.g. a dot, a cross, a square, or the like, by means of text, and/or by an overlay indicating the endoscope position.

In some embodiments, the processing unit is further configured to:
store at least one previous endoscope position and display on the model of the human airways the at least one previous endoscope position.

This, in turn, allows the image processing device to indicate to the medical personnel where the endoscope previously has been in the airways, again allowing the medical personnel to easily and quickly navigate the endoscope to other parts of the airways during the examination.

Where it is determined that the anatomic reference position corresponds to a previous position, this may be displayed.

The previous endoscope position may be indicated on the display in any known way. Where the determined endoscope position is indicated in combination with the previous endoscope position, the previous endoscope position may be indicated differently from the determined endoscope position. For example, the previous position may be indicated by a part of the human airways, in which the endoscope position was previously located, changing colour, flashing, being highlighted, or the like, by a marking arranged at a position in the model, such as a black and white or coloured shape, e.g. a dot, a cross, a square, or the like, by means of text, and/or by an overlay indicating the endoscope position. The marking colour, flashing frequency, highlight, and/or the shape of the marking may be different from those that indicate the determined endoscope position in the model.

In some embodiments, the image processing device further comprises input means for receiving a predetermined desired position in the lung tree, the processing unit being further configured to:
indicate on the model of the human airways the predetermined desired position.

The predetermined desired position may be a specific part or a specific area in a lung tree, such as one or more specific bronchi, one or more specific bronchioles, and/or one or more specific alveoli. The predetermined desired position may be a part, in which an examination, e.g. for abnormalities, is to take place.

The predetermined desired position may be input to the image processing device in any known way, such as by means of one or more of a touchscreen, a keyboard, a pointing device, a computer mouse, and/or automatically or manually based on information from a CT scan or MR scan output.

The indication on the model may be performed in a manner similar to the indications described with respect to the previous endoscope position and/or the determined endoscope position.

In some embodiments, the processing unit is further configured to:
determine a route to the predetermined desired position, the route comprising one or more predetermined desired endoscope positions,
determine whether the updated endoscope position corresponds to at least one of the one or more predetermined desired endoscope positions, and
where it is determined that the updated endoscope position does not correspond to at least one of the one or more predetermined desired endoscope positions, provide an indication on the model that the updated endoscope position does not correspond to at least one of the one or more predetermined desired endoscope positions.

Thereby, the medical personnel may be provided with a suggested route to the predetermined desired position, allowing for an easy navigation of the endoscope as well as a potentially time-reduced examination as wrong navigations with the endoscope can be avoided or indicated as soon as the wrong navigation has occurred.

In some embodiments, the route may be determined from the entry of the human airways and/or from the updated anatomic reference position. In some embodiments, the route may be a direct route to the predetermined desired position. Additionally, or alternatively, the route may be determined as a route via one or more reference positions. The route may be updated after each update of the endoscope position. Where the route is updated after each update of the endoscope position, a turn-by-turn navigation-like functionality may be provided, e.g. such that the medical personnel may be provided with information of how to navigate the endoscope when a furcation occurs in the human airways. The route may be updated in response to the determination that the endoscope position is not on the route, e.g. does not correspond to or is not equal to one of the predetermined desired positions.

In some embodiments, the processing unit may determine the route based on an algorithm therefor. Alternatively or additionally, the route may be determined based on trial-and-error of different potential routes. In some embodiments, the route is determined by the machine learning data architecture.

The one or more predetermined desired endoscope positions may be one or more predetermined intermediate endoscope positions. In some embodiments the one or more predetermined desired endoscope positions each correspond to an endoscope position in the model.

In some embodiments, the route may comprise one or more previous positions of the endoscope.

In some embodiments, the machine learning data architecture is trained by:
determining a plurality of anatomic reference positions of the body cavity,
obtaining a training dataset for each of the plurality of anatomic reference positions based on a plurality of endoscope images,
training the machine learning model using said training dataset.

The training dataset may comprise a plurality of images. The training dataset may be updated to include a plurality of the images from the stream of recorded images.

The body cavity may be the human airways and/or a lung tree.

A second aspect of the present disclosure relates to an endoscope system comprising an endoscope and an image processing device according to the first aspect of this disclosure, wherein the endoscope system has an image capturing device, and wherein the processing unit of the image processing device is operationally connectable to said image capturing unit of the endoscope.

The endoscope may be an endoscope comprising one or more of a handle at a proximal end thereof, an insertion tube extending in a proximal-distal direction, a bending section, and/or a tip part at a distal end of the endoscope, a proximal end of the tip part potentially being connected to a distal end of a bending section. The image capturing device may be connected to the image processing unit in a wired and/or in a wireless manner.

The processing unit may be configured to receive one or more images from the endoscope image capturing device. The one or more images may be a stream of recorded images.

In some embodiments, the endoscope system further comprises a display unit, wherein the display unit is operationally connectable to the image processing device, and wherein the display unit is configured to display at least a view of the model of the human airways.

The display unit may be configured to display the model and/or display a video stream from the image capturing device. The display unit may moreover be configured to display the stream of recorded images. The display may be any known display or monitor type, potentially as described with respect to the first aspect of this disclosure.

A third aspect of the present disclosure relates to a display unit comprising an image processing device according to the first aspect of this disclosure.

A fourth aspect of the present disclosure relates to a computer program product comprising program code means configured to cause at least a processing unit of an image processing device to perform the steps of the first aspect of this disclosure, when the program code means are executed on the image processing device.

The different aspects of the present disclosure can be implemented in different ways including image processing devices, display units, endoscope systems, and compute program products described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependant claims. Furthermore, it will be appreciated that embodiments described in connection with one of the aspects described herein may equally be applied to the other aspects.

### Brief description of the drawings

The tip part assemblies and methods will now be described in greater detail based on non-limiting exemplary embodiments and with reference to the drawings, on which:
FIG. 1a shows a perspective view of an endoscope in which a tip part assembly according to the present disclosure is implemented,
FIG. 1b shows a perspective view of a display unit to which the endoscope of FIG. 1a is connected,
FIG. 2 shows a flow chart of steps of a processing unit of an image processing device according to an embodiment of the disclosure,
FIG. 3 shows a flow chart of steps of a processing unit of an image processing device according to an embodiment of the disclosure,
FIG. 4a shows a view of a schematic model of a display unit according to an embodiment of the disclosure,
FIG. 4b shows a view of a schematic model of a display unit according to an embodiment of the disclosure,
FIG. 5 shows a schematic drawing of an endoscope system according to an embodiment of the disclosure, and
FIG. 6 shows a view of an image of a display unit according to an embodiment of the disclosure.

Similar reference numerals are used for similar elements across the various embodiments and figures described herein.

### Detailed description

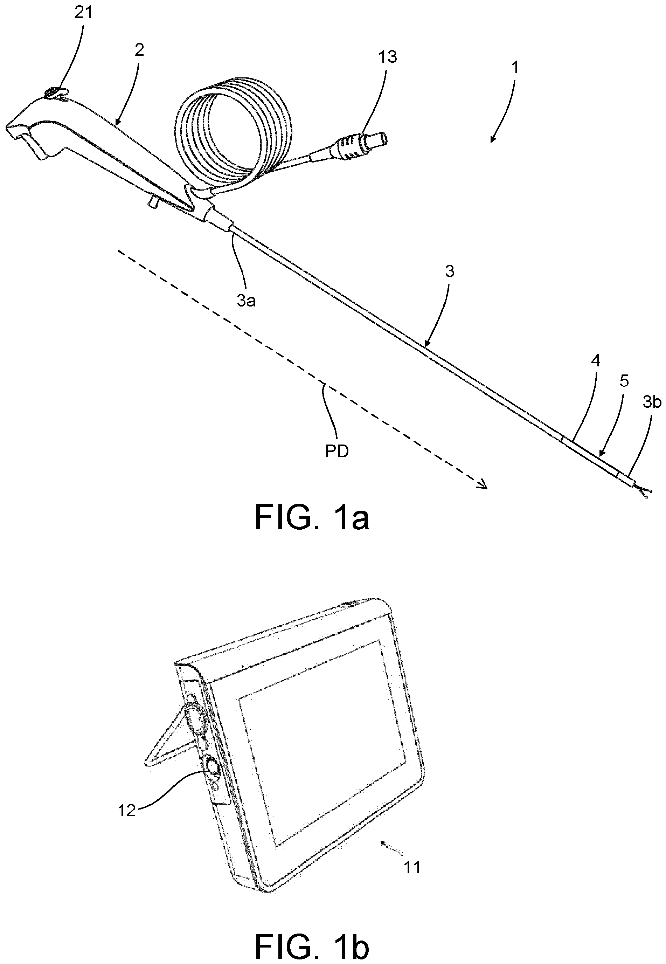

Referring first to FIG. 1a, an endoscope 1 is shown. The endoscope is disposable, and not intended to be cleaned and reused. The endoscope 1 comprises an elongated insertion tube 3. At the proximal end 3a of the insertion tube 3 an operating handle 2 is arranged. The operating handle 2 has a control lever 21 for manoeuvring a tip part assembly 5 at the distal end 3b of the insertion tube 3 by means of a steering wire. A camera assembly 6 is positioned in the tip part 5 and is configured to transmit an image signal through a monitor cable 13 of the endoscope 1 to a monitor 11.

In FIG. 1b, a display unit comprising a monitor 11 is shown. The monitor 11 may allow an operator to view an image captured by the camera assembly 6 of the endoscope 1. The monitor 11 comprises a cable socket 12 to which a monitor cable 13 of the endoscope 1 can be connected to establish a signal communication between the camera assembly 6 of the endoscope 1 and the monitor 11.

The monitor 11 shown in FIG. 1b is further configured to display a view of the model. The display unit further comprises an image processing device.

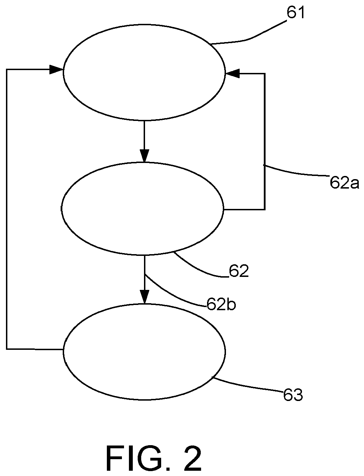

FIG. 2 shows a flow chart of steps of a processing unit of an image processing device according to an embodiment of the disclosure. The steps of the flow chart are implemented such that the processing unit is configured to carry out these steps.

In the first step 61, a stream of images is obtained from an image capture device, such as a camera unit, of an endoscope. In step 62, an image from the stream of images is analysed to determine whether an anatomic reference position has been reached. In other embodiments, a plurality of images from the stream of images may be analysed sequentially or simultaneously in step 62.

Where it is determined in step 62 that an anatomic reference position has not been reached, the processing unit returns to step 61 as indicated by decision 62a. The step 61 of obtaining images from the image capturing unit as well as the step 62 of analysing the images may be carried out simultaneously and/or may be carried out sequentially.

An anatomic reference position is a position, at which a furcation occurs. In step 62, the processing unit determines whether an anatomic reference position has been reached by determining whether a furcation is seen in an image from the obtained stream of images using a machine learning data architecture. The machine learning data architecture is trained to detect a furcation in images from an endoscope. In other embodiments, the anatomic reference positions may be other positions, potentially showing features different from or similar to that of a furcation.

Where it is determined in step 62 that an anatomic reference position has been reached, the processing unit is configured to proceed to step 63 as indicated by decision 62b. In step 63, an endoscope position is updated in a model of the human airways based on the determined anatomic reference position. This may comprise generating an endoscope position and/or removing a previous endoscope position and inserting a new endoscope position.

The endoscope position is determined in step 63 as one in a plurality of predetermined positions present in the model, based on the determination that an anatomic reference position has been reached and a previous position, e.g. previously determined by the processing unit.

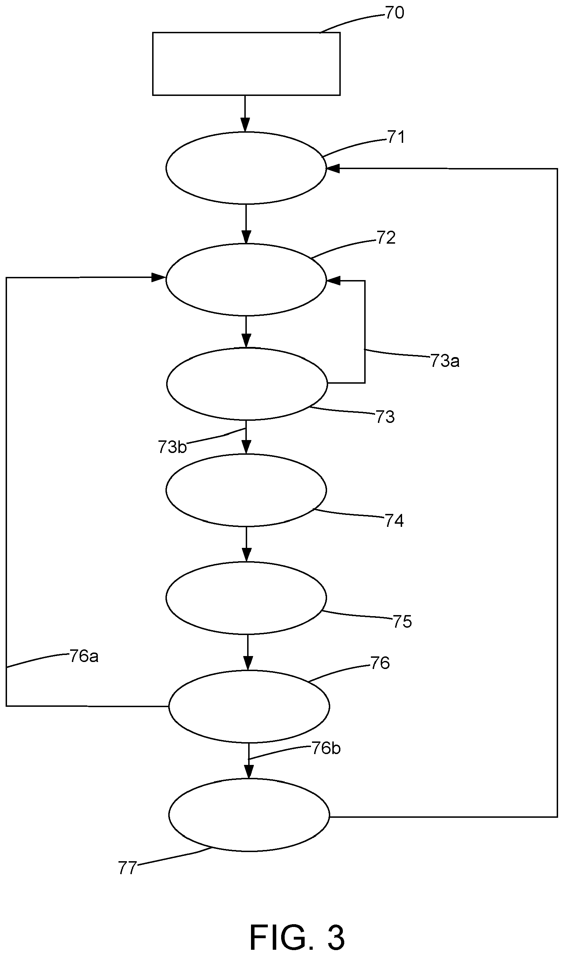

FIG. 3 shows a flow chart of steps of a processing unit of an image processing device according to an embodiment of the disclosure.

In step 70 of the flow chart shown in FIG. 3, a model of the human airways is provided. The model provided in step 70 is a generic, well-known model including an overall structure of the human airways. The model of the human airways comprises a trachea, bronchi, i.e. left and right primary, secondary, and tertiary bronchi, and a number of bronchioles. In some embodiments, the model generated in step 70 may be generated from or based on an output from an MR scan and/or a CT scan of the human airways, potentially from a specific patient.

In step 70, a predetermined desired position on the model is furthermore input. The predetermined desired position can, e.g., be a bronchus and/or a bronchiole.

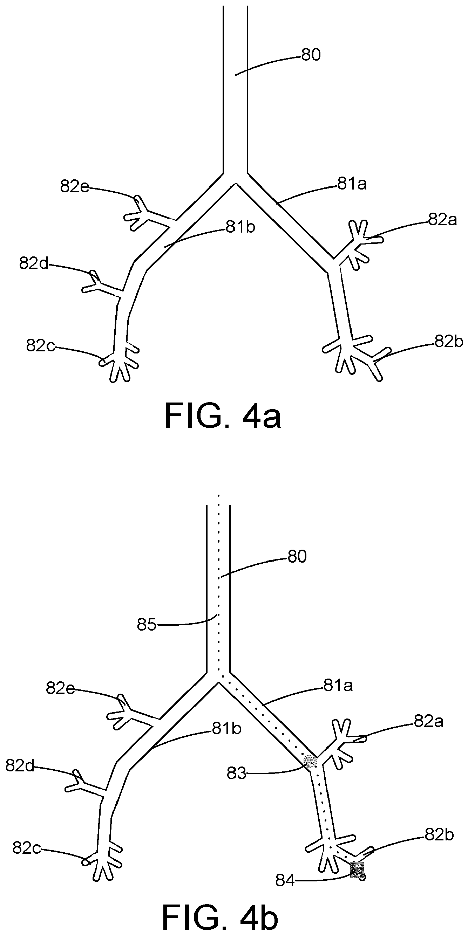

In step 70 of the flow chart shown in FIG. 3, a view of the model is furthermore displayed on a display unit. The display unit may be a display unit as shown in and described with respect to FIG. 1b. The view of the model is an overall structure of the human airways. In some embodiments, the view may be a view as shown and described with respect to FIG. 4a and/or a view as shown and described with respect to FIG. 4b. In step 70, an initial position of the endoscope is furthermore indicated on the view of the model. The initial position may be in an upper portion of trachea as shown in the view of the model.

In step 71, a route from a starting point, e.g. an entry into the human airways, and/or a part of the human airways such as the trachea, to the predetermined desired position is determined throughout the model. The route may comprise a number of predetermined positions in the human airways, potentially corresponding to potential endoscope positions and/or to anatomic reference positions. In some embodiments, a plurality of predetermined desired positions may be provided, and individual routes and/or a total route may be provided.

In step 71, the determined route is furthermore shown in the view of the model displayed in step 70. The route may be shown by a marking, e.g. as illustrated in the model view of FIG. 4b.

In step 72, a stream of images is obtained from an image capture device, such as a camera unit, of an endoscope. The endoscope may be an endoscope as shown in and described with reference to FIG. 1a. Step 72 may be performed simultaneously with step 71 and/or step 70.

In step 73, an image from the stream of images is analysed to determine whether an anatomic reference position has been reached. In step 73 the analysis is carried out using a machine learning data architecture. In other embodiments, a plurality of images from the stream of images may be analysed sequentially or simultaneously in step 73. In step 73, either a decision 73a is taken that it is determined that an anatomic reference position has not been reached, or a decision 73b is taken that it is determined that an anatomic reference position has been reached.

Where decision 73a is taken, the processing unit is configured to return to step 72, in which a stream of images is obtained from an endoscope, i.e. from an image capture unit of an endoscope. Step 72 and step 73 may be performed simultaneously or sequentially, and a stream of images may be obtained whilst the processing unit is determining whether an anatomic reference position has been reached. Steps 72, 73 and 73a corresponds to steps 61, 62, and 62a, respectively, of the flow chart shown in FIG. 2.

Where decision 73b is taken, the processing unit goes to step 74, corresponding to step 63 of the flow chart shown in FIG. 2. In step 74, an endoscope position is updated in the model of the human airways based on the determined anatomic reference position and a previous position of the endoscope. In other embodiments, the endoscope position may be updated in various alternative ways as described with reference to FIG. 2.

In step 75, the updated endoscope position is shown in the view of the model generated in step 71. The updated endoscope position is shown by a marker arranged at a position in the model corresponding to the updated endoscope position. The updated endoscope position replaces the previous endoscope position in the model. Alternatively, one or more previous positions may remain shown on the view of the model, potentially indicated such that the updated position is visually distinguishable from the previous position(s). For instance, markers indicating a previous endoscope position may be altered to be of a different type or colour than the marker indicating an updated endoscope position.

In step 75, the updated position may furthermore be stored. The updated position is stored in a local non-transitory storage of the image processing device. The updated position may alternatively or subsequently be transmitted to an external non-transitory storage.

A number of images from the stream of images, in which an anatomic reference position was detected in step 73, may furthermore be stored in step 75 and the image(s) from the stream of images. The images may be stored with a reference to the updated reference position in local non-transitory storage and/or in external non-transitory storage. The stored images may furthermore be used by the machine learning data architecture, e.g. to improve the detection of anatomic reference positions. For example, one or more of the stored image(s) and/or the reached anatomic reference position may be introduced into a dataset of the machine learning data architecture.

In step 76, the processing unit determines whether the updated endoscope position is on the route determined in step 70 by determining whether the updated endoscope position corresponds to one of the predetermined positions in the human airways included in the route. In step 76, two decisions may be taken, where one decision 76a is that the updated endoscope position is on the route, and the other decision 76b is that the updated endoscope position is not on the determined route.

Where decision 76a is taken, the processing unit returns to step 72.

Where decision 76b is taken, the processing unit proceeds to step 77, in which an indication that the updated position is not on the route determined in step 71, is provided to a user, i.e. medical personnel. The indication may be a visual indication on a display unit and/or on the view of the model, and/or may be an auditory cue, such as a sound played back to the user, or the like.

Subsequent to providing the indication in step 77, the processing unit returns to step 71 and determines a new route to the predetermined desired position from the updated endoscope position.

It should be noted that it will be understood that steps 72 and 73 may run in parallel with steps 71, 74-77 and/or that decision 73b may interrupt steps 74-77 and 71.

FIG. 4a shows a view of a schematic model of a display unit according to an embodiment of the disclosure.

The view of the schematic model may be generated in step 70 of the flow chart of FIG. 3 and/or may be displayed on the display unit shown in and described with reference to FIG. 1b.

In the view of FIG. 4a, a schematic model of the human airways is shown. The view shown in FIG. 4a is not necessarily in scale and the relative size of individual parts or elements therein does not necessarily correspond to the relative sizes of the parts or elements of the human airways which they model. In FIG. 4a, the view illustrates a trachea 80, a left primary bronchus 81a and a right primary bronchus 81b. The model moreover shows secondary bronchi as well as some bronchioles 82a-82e.

The view of the model shown in FIG. 4a may in some embodiments be more or less detailed. The view shown in FIG. 4a may be displayed on the display unit shown in and described with respect to FIG. 1b.

FIG. 4b shows a view of a schematic model of a display unit according to an embodiment of the disclosure.

Similar to the view shown in FIG. 4a, the view of the model in FIG. 4b illustrates a trachea 80, left and right bronchi 81a, 81b, respectively, and groups of bronchioles 82a-82e.

In the view of the schematic model shown in FIG. 4b, an estimated position 83 of the endoscope is shown. The estimated endoscope position 83 is indicated by a dot arranged at the position in the model substantially corresponding to and representing the position of the endoscope in the human airways. It should be noted that the dot need not show an exact real-time position of the endoscope position but may show an approximated position or an area or part of the human airways, in which the endoscope is estimated to be positioned.

In the view of the schematic model shown in FIG. 4b, a predetermined desired position 84 is furthermore indicated in one of the bronchioles of the group of bronchioles 82b.

In the view of FIG. 4b, a route 85 to the predetermined position 84 from an initial endoscope position, i.e. the upper end of the trachea, is furthermore shown. As seen in FIG. 4b, the estimated endoscope position 83 is on the route 85 to the predetermined position 84. In a case where the estimated endoscope position is not on the route 85 to the predetermined position 84, this may be indicated in the view of FIG. 4b. Alternatively or additionally, this may be indicated in another view and/or in another way. For instance, it may be determined that an updated endoscope position is not on the route, if in the view of FIG. 4b a next updated endoscope position is in the path to the bronchioles 82a rather than on the route 85.

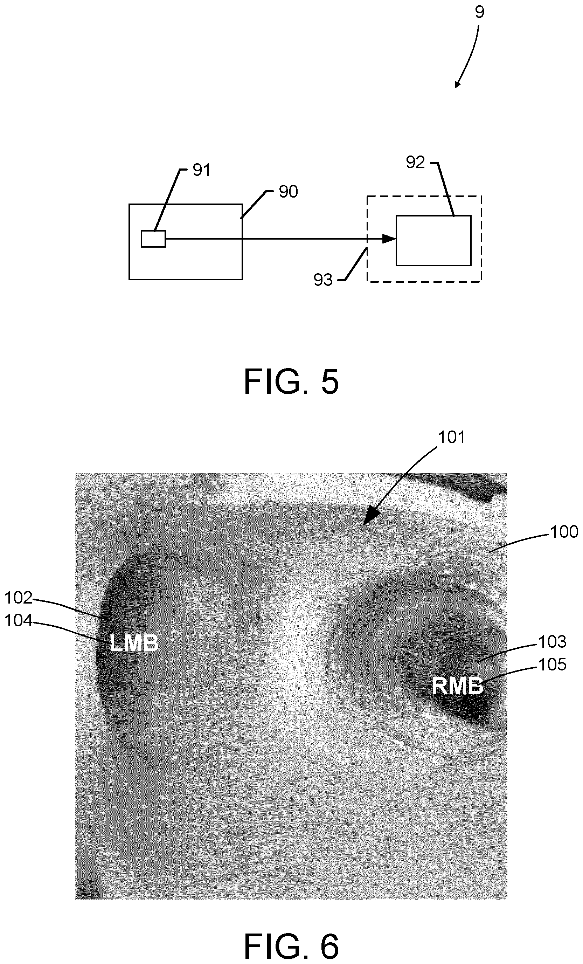

FIG. 5 shows a schematic drawing of an endoscope system 9 according to an embodiment of the disclosure. The endoscope system 9 comprises an endoscope 90, an image processing device 92 having a processing unit. The endoscope 90 has an image capturing device 91 and the processing unit of the image processing device 92 is operationally connectable to the image capturing device of the endoscope 91. In this embodiment, the image processing device 92 is integrated in a display unit 93. In this embodiment, the image processing device 92 is configured to estimate a position of the endoscope 90 in a model of the human airways using a machine learning data architecture trained to determine a set of anatomic reference positions, said image processing device comprising a processing unit operationally connectable to an image capturing device of the endoscope. In this embodiment, the processing unit of the image processing device 92 is configured to:
obtain a stream of recorded images;
continuously analyse the recorded images of the stream of recorded images using the machine learning data architecture to determine if an anatomic reference position of a subset of anatomic reference positions, from the set of anatomic reference positions, has been reached; and
where it is determined that the anatomic reference position has been reached, update the endoscope position based on the anatomic reference position.

FIG. 6 shows a view of an image 100 of a display unit according to an embodiment of the disclosure. The image 100 is an image from a stream of recorded images. The stream of recorded images is recorded by a camera module of an endoscope. The image 100 has been analysed by an image processing device.

The image 100 shows a branching, i.e. a bifurcation 101, of the trachea into a left primary bronchus 102 and a right primary bronchus 103. The bifurcation 101 is a predetermined anatomic reference position, and the image processing device determines based on the image 100 that the bifurcation 101 has been reached and updates the position of the endoscope in the model of the human airways (not shown in FIG. 6). Where a view of a schematic model, as e.g. shown in FIGs. 4a and 4b, is provided, the image processing device may update a view of the estimated endoscope position on this view.

In the image 100, the image processing device determines the two branches of the bifurcation 101 as the left main bronchus 102 and the right main bronchus 103 using the machine learning data architecture of the image processing device. The image processing device provides a first overlay 104 on the image 100 indicating to the operator, e.g. the medical personnel, the left main bronchus 102 and a second overlay 105 indicating to the operator the right main bronchus 103. The first 104 and second overlays 105 are provided on the screen in response to the user pushing a button (not shown). The first 104 and second overlays 105 may be removed by pushing the button again.

Where the operator navigates the endoscope into either the left main bronchus 102 or the right main bronchus 103, it is determined by the image processing device which of left 102 and right main bronchus 103, the endoscope has entered. The image processing device updates the estimated endoscope position based on the determined one of the left 102 or right main bronchus 103, the endoscope has entered. When a subsequent branching is encountered, the image processing device determines the location of the branching in the model of the human airways based on the information regarding which of the main bronchi 102, 103, the endoscope has entered.

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. An image processing device for estimating a position of an endoscope in a model of the human airways using a machine learning data architecture trained to determine a set of anatomic reference positions, said image processing device comprising a processing unit operationally connectable to an image capturing device of the endoscope, wherein the processing unit is configured to:
obtain a stream of recorded images;
continuously analyse the recorded images of the stream of recorded images using the machine learning data architecture to determine if an anatomic reference position of a subset of anatomic reference positions, from the set of anatomic reference positions, has been reached; and
where it is determined that the anatomic reference position has been reached, update the endoscope position based on the anatomic reference position.

2. The image processing device of claim 1, wherein the subset comprises a plurality of anatomic reference positions.

3. The image processing device of claim 1 or 2, wherein the processing unit is further configured to:
where it is determined that the anatomic reference position has been reached, update the subset of anatomic reference positions.

4. The image processing device of claim 3, wherein the updated subset of anatomic reference positions comprises at least one anatomic reference positions from the subset of anatomic reference positions.

5. The image processing device according to any one of the preceding claims, wherein the anatomic reference position is a branching structure comprising a plurality of branches, and wherein the image processing device is further configured to:
determine which branch from the plurality of branches the endoscope enters; and
update the endoscope position based on the determined branch.

6. The image processing device according to any one of the preceding claims, wherein the processing unit is further configured to:
where it is determined that the anatomic reference position has been reached, storing a part of the stream of recorded images.

7. The image processing device according to any one of the preceding claims, wherein the processing unit is further configured to:
prior to the step of updating the subset of anatomic reference positions, generate the model of the human airways, and/or
subsequent to the step of updating the subset of anatomic reference positions, update the model of the human airways based on the reached anatomic reference position and/or an anatomic reference position of the updated subset of anatomic reference positions.

8. The image processing device according to claim 7, wherein the model of the human airways is a schematic model of the human airways, preferably generated based on images from a magnetic resonance (MR) scan output and/or a computed tomography (CT) scan output.

9. The image processing device according to any one of the preceding claims, wherein the processing unit is further configured to:
subsequent to the step of updating the endoscope position, perform a mapping of the endoscope position to the model of the human airways and display the endoscope position on a view of the model of the human airways.

10. The image processing device according to any one of the preceding claims, wherein the processing unit is further configured to:
store at least one previous endoscope position and display on the model of the human airways the at least one previous endoscope position.

11. The image processing device according to any one of the preceding claims further comprising input means for receiving a predetermined desired position in the lung tree, the processing unit being further configured to:
indicate on the model of the human airways the predetermined desired position.

12. The image processing device according to claim 11, wherein the processing unit is further configured to:
determine a route to the predetermined desired position, the route comprising one or more predetermined desired endoscope positions,
determine whether the updated endoscope position corresponds to at least one of the one or more predetermined desired endoscope positions, and
where it is determined that the updated endoscope position does not correspond to at least one of the one or more predetermined desired endoscope positions, provide an indication on the model that the updated endoscope position does not correspond to at least one of the one or more predetermined desired endoscope positions.

13. The image processing device according to any one of the preceding claims, wherein the machine learning data architecture is trained by:
determining a plurality of anatomic reference positions of the body cavity,
obtaining a training dataset for each of the plurality of anatomic reference positions based on a plurality of endoscope images,
training the machine learning model using said training dataset.

14. An endoscope system comprising an endoscope and an image processing device according to any one of the preceding claims, wherein the endoscope system has an image capturing device, and wherein the processing unit of the image processing device is operationally connectable to said image capturing unit of the endoscope.

15. An endoscope system according to claim 14 further comprising a display unit, wherein the display unit is operationally connectable to the image processing device, and wherein the display unit is configured to display at least a view of the model of the human airways.

16. A display unit comprising an image processing device according to any one of claims 1-13.

17. A computer program product comprising program code means configured to cause at least a processing unit of an image processing device to perform the steps of any one of claims 1-13, when the program code means are executed on the image processing device.
